Europäisches Patentamt

(19) European Patent Office

Office européen des brevets

(11) Publication number: **0 037 612**

**B1**

(12) **EUROPEAN PATENT SPECIFICATION**

(45) Date of publication of patent specification: **24.07.85**

(21) Application number: **81200374.7**

(22) Date of filing: **02.04.81**

(51) Int. Cl.⁴: **C 05 F 13/00**

(54) **A method for the anaerobic conversion of solid organic material.**

(30) Priority: **03.04.80 NL 8001997**
**02.12.80 NL 8006567**

(43) Date of publication of application:
**14.10.81 Bulletin 81/41**

(45) Publication of the grant of the patent:
**24.07.85 Bulletin 85/30**

(84) Designated Contracting States:
**AT BE CH DE FR GB IT LI LU NL SE**

(56) References cited:
**none**

(73) Proprietor: **INSTITUUT VOOR BEWARING EN VERWERKING VAN LANDBOUWPRODUKTEN**
**Bornsesteeg 59 P.O. Box 18**
**NL-6700 AA Wageningen (NL)**

(72) Inventor: **Rijkens, Berend Abraham**
**Lyceumlaan 15**
**NL-3707 EA Zeist (NL)**

(74) Representative: **van der Beek, George Frans et al**
**Nederlandsch Octrooibureau Johan de Wittlaan 15 P.O. Box 29720**
**NL-2502 LS 's-Gravenhage (NL)**

## Description

The invention relates to a method for the anaerobic digestion of solid organic material, originating from plants or animals or both,

In the "Winkler Prins Technische Encyclopedie" (1), page 119 (1975) is stated that the so-called aerobic digestion is a long standing method for converting a solid organic waste into compost. The method for forming a compost comprises the microbiological digestion of organic compounds that may be attacked easily under aerobic conditions. The compost forming method usually applied is the open air or land fill method in which the waste material is disposed off by forming heaps. During the treatment these heaps are moistened and turned over several times by means of an excavator or a grabbing crane.

The latter method is labour, time and space consuming, so that the expenses are considerable. Other drawbacks of the method are, that in the aerobic digestion valuable organic material is converted partially into worthless carbon dioxide and water on the one hand and on the other hand conditions are required to avoid pollution of the ground water. "$H_2O$ Tijdschrift vapor watervoorziening en afvalwaterbehandeling" (Periodical for watersupply and wastewater treatment) (22) 531 (1977) discloses that aqueous suspensions and solutions of organic waste and inorganic salts that have low viscosity and a low solids contents may be subjected to an anaerobic fermentation. According to this disclosure a part of the polysaccharides present (e.g. cellulose) is hydrolized to soluble monomers in a preceding step in an open pond under the influence of optionally anaerobic bacteria under nonstrictly anaerobic conditions, subsequently said soluble monomers are converted into fatty acids. Then, in a reactor under anaerobic conditions methane is formed from said fatty acids. Such a method cannot be applied for the digestion of solid organic waste to form compost.

An improvement of said anaerobic fermentation of aqueous suspensions and solutions, that have a low viscosity and a low solids content may be obtained by performing the preceding step in a tank under anaerobic conditions, but even then this method, disclosed in "$H_2O$" (10), 296 (1977) will not be suitable for the digestion of solid organic waste.

In French Patent Specification 2.324.581 a process for the anaerobic fermentation of sludges from sewage treatment plants, night soil and domestic animal droppings having a solids concentration of up to 5 weight %, is disclosed. In case the material to be treated has too large dimensions it is subjected to a pulverizing operation in order to obtain particles that may be used for the preparation of an aqueous slurry, since it is imperatively required that the starting material is an aqueous slurry having a low percentage of solids. In a first step of the anaerobic fermentation water-soluble products and low molecular weight fatty acids are formed. When the acid-forming fermentation has been completed, the supernatant liquid is fed from the acid reactor to a methane reactor. In order to promote the fermentation proper micro-organisms are added and in order to control the pH in the reactor acids respectively alkaline materials are added.

In the embodiments of the anaerobic fermentation of aqueous suspensions and solutions of organic material that have a low viscosity and a low solids content, in the methane reactor a bacteria rich sludge, preferably a grainy product is separated from the effluent and the gas.

If one desired to generate methane in a single step from the solid organic material instead of from aqueous suspensions or solutions of organic material that have a low viscosity and a low solids content it appears that the reaction rate is very low. The reason therefore is, that soon that high an acidity is reached that the medium will have a strongly propagation inhibiting effect for the acid generating microorganisms as well as for the methane generating microorganisms. Consequently, there is no further acid generation and no methane generation either, but there occurs a generation of bad smell ($H_2S$, other sulfur compounds and $NH_3$) (vide "Journal of the environmental engineering division", June 1978, pages 415—422).

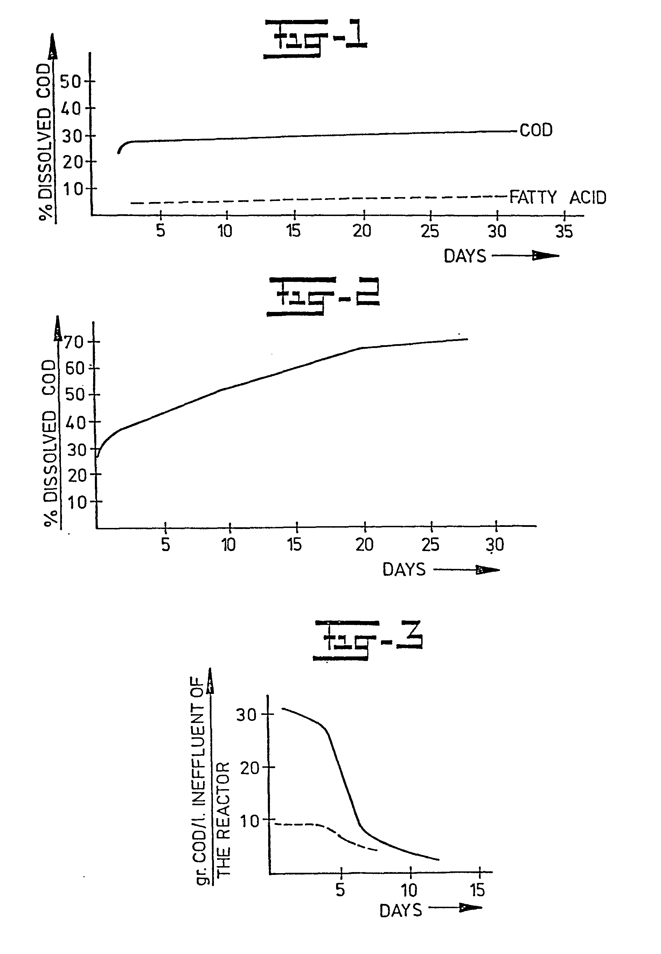

The curves of the percentage of dissolved COD and of the acid generation vs time in such a conversion have been shown in a graph in Fig. 1, wherein the interrupted line indicates the dissolved fatty acid-COD content.

From said Figure it is apparent that in such a case more than 25% of the maximum soluble COD is already dissolved from an inocculated mixture of straw+beet pulp+dried cow dung after a pre-acidolysis for 3 days. The COD then comprises 14% of fatty acid-COD. It is apparent that after a period of 30 days this has been increased to about 25%. No methane gas is formed in that stage.

Attempts have also been made to perform the anaerobic fermentation of a solid organic waste according to the above described methods for suspensions or solutions of organic material having a low viscosity and a low solids content by first grinding the dry material then dispersing the ground material in water and subsequently fermenting it anaerobically. However, it has been found that the dry solids content of such a slurry may not exceed 5 percent by weight.

Furthermore the publication PB—258499 of the U.S. Department of Commerce dated August 1976, page 30 aff. discloses on page 36 a method wherein a slurry having a solids content of 12% by weight is prepared by using the effluent of the methanereactor for the preparation of the slurry of the solid waste.

Likewise such a method is disclosed in US—A—4,022,665, wherein waste, e.g. manure, municipal refuse, raw sewage, primary sludge, activated sludge or biomass, if desired having been subjected to a hydrolyzing treatment, is fed continuously to the first step of the reaction in an amount of from 16 to 160 g/l, from which material under agitation and under anaerobic conditions low molecular fatty acids are formed.

Subsequently said liquid mixture that contains fatty acids and other break-down products is fed intermittently or continuously in an amount of from 1.6 to 8 g. of organic material/l/day from the first (acidifying) step of the reaction to at least one second (methane-forming) step, wherein said acidic liquid mixture under agitation and under anaerobic conditions is reacted into carbon dioxide and methane. The remaining part of said liquid mixture that may consist of liquid and/or solid material, is, if desired after having been subjected to a hydrolyzing treatment, recycled to the first step of the reaction to be subjected again to the whole process.

It was found now that a much more efficient anaerobic conversion of solid organic material may be achieved by

a) simultaneously decomposing said solid organic waste under anaerobic conditions in a primary reaction space provided with a strainer while

b) rinsing the solid organic waste from step (a) with water that may be polluted with soluble and/or insoluble inorganic and/or organic materials originating from plants and/or animals, to substantially dissolved and remove soluble organic and inorganic substances and the water soluble fatty acids formed by the decomposition;

c) feeding the liquid obtained in step (b) substantially free from undissolved solid organic waste from the primary reaction space into an auxiliary reactor;

d) leaving the remaining compost behind in the primary reaction space;

e) under anaerobic conditions in the auxiliary reactor, converting the dissolved organic material in the aqueous solution into a mixture of carbon dioxide and methane gas; and

f) removing the remaining compost from the primary reaction space.

Important differences between the present process and the process of US—A—4,022,665 are:

1) in the process of the invention the waste is not fed continuously to the reaction space;
2) in the process of the invention the water soluble fatty acids and other water soluble organic materials generated under anaerobic conditions in the first step as well as inorganic materials present therein are removed substantially completely from the reaction mass by rinsing with an aqueous liquid. Consequently, the water insoluble materials remain in the reaction space. Due to said condition it is avoided that in the reaction spaced the pH will become too low and/or the concentration of other inhibiting products will become that high that the growth of the microbial flora is inhibited by too high a concentration of inhibiting substances or too low a pH-value of the medium. Moreover it is avoided that the materials that have not been broken down to liquid products, e.g. cellulose containing materials, will be fed to the auxiliary reactor, will be broken down and converted in said reactor and will affect the production of methane;
3) according to a preferred embodiment of the method according to the invention, after separation of water and gas in the auxiliary reactor just the aqueous phase is used for rinsing the mass present in the reaction space;
4) in the process according to the invention a stabilized compost is formed in the primary reaction space, which compost is removed from said reaction space when the conversion has been completed. In the known method never a stabilized mass is obtained in the reaction space.

Draw-backs of the methods known from PB—258499 of U.S. Department of Commerce loc. cit. and US—A—4.022.665 are that there will be consumed energy for grinding the solid material and due to the presence of many colloidal materials in said slurries the use of very expensive reactors is required.

When applying the method according to the invention the water rinse is effective to decrease the concentration of inhibiting substances in the reactor and to avoid a decrease of the pH-value. In this way it is prevented that the propagation of the microbial flora is inhibited by too high a concentration of inhibiting substances or to low a pH-value of the medium.

Although rinsing may be performed with freshly supplied water it is preferred to use the effluent from the auxiliary reactor after conversion of the fatty acids present therein by recirculating it as the rinse liquid for the reactor.

The said anaerobic fermentations may be performed within the mesophilic temperature range (5—45°C) and within the thermophilic temperature range (25—70°C).

As examples of solid organic materials to be digested there may be mentioned domestic waste, straw, vegetable waste, leaves, rinse, peels, skins, peelings and grass and also other organic materials which may have a moisture content of about 10—60 weight %. More particularly the process is used for wet perishable and evil-smelling animal and/or vegetable waste that should be kept in a closed space.

In the method according to the invention also so called "energy farming plants", e.g. kelp, may be used as the organic starting material.

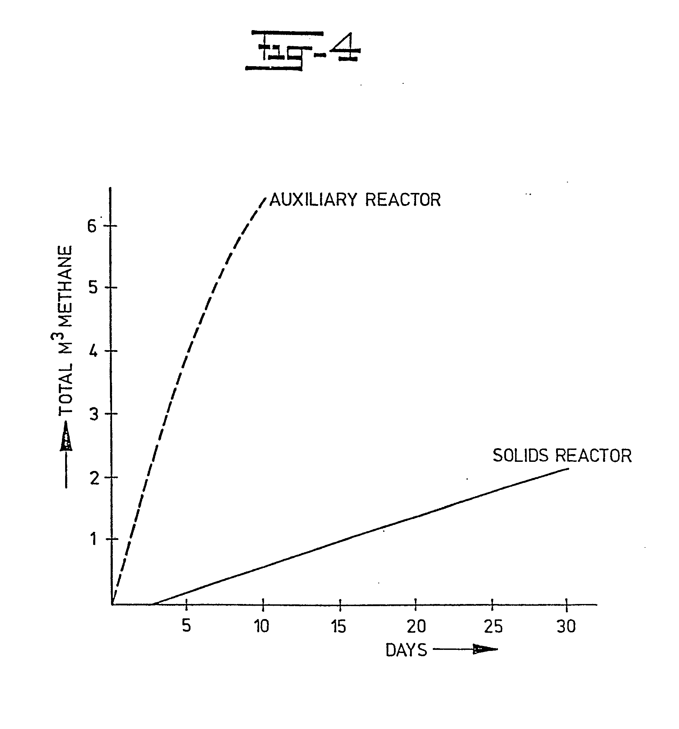

It has been found that in case of a semi-stagnant operation, that is to say that a drainage and a refreshment of the liquid of the reaction space is performed once a day a COD-solution curve as shown e.g. in Fig. 2 is achieved. In this case the dissolved COD comprises about 30% of fatty acid-COD after 2 days and about 35% of fatty acid-COD after 7 days.

When applying a continuous rinsing there will be obtained a COD-curve in the effluent of the reactor vs. time as shown in Fig. 3.

In this case the effluent from the auxiliary reactor is used as the rinsing agent for the (primary) reaction space.

Hence it appears from the solid line that after 10 days only little COD is dissolved from the reaction space and from the interrupted line indicating the fatty acid-COD content, that this soluble COD consists almost entirely of fatty acids.

The methane generation in such a conversion within the mesophilic temperature range has been indicated in Fig. 4. The solid line pertains to the gas generation in the (primary) reaction space. The dot-and-dash-line represents the methane generation in the auxiliary reactor.

After 10 days about 75 percent of the fermentable solid organic material has been digested and discharged in the form of a solution.

It takes more than 30 days to decompose the remaining 25% of fermentable organic material.

When carrying out the method according to the invention in practice it may appear to be appropriate to use more than one (primary) reaction space for each auxiliary reactor. When applying this way of operation it is possible to supply an influent of a homogeneous composition to the auxiliary reactor so that an optimal performance of the auxiliary reactor may be obtained.

In order to promote the decomposition of particular materials in the primary reaction space, auxiliary materials may be added.

Thus, in order to promote hydrolysis of cellulose, cellulase and/or a cellulase generating culture of microorganisms, viz. bacteria and fungi may be added to the cellulose containing material that has to be decomposed.

Other hydrolysis promoting additives are diastase or amylase to decompose starch, pectinase for the hydrolization of pectin and inulase for the hydrolysis of inuline.

The primary reaction space may be used as a bad smell free storage yard. If waste is delivered intermittently into the reaction space, this will not start to develop a bad smell since said reaction space is fully sealed, while the acids present and the acids produced have a preserving effect.

At any time desired the reaction space may be put into operation by rinsing it with an aqueous liquid. Said way of using said reaction space provides an important advantage comparative to the old methods, wherein in the week-ends provisions are required to work up the waste stored in an efficient sequence.

Hardly any elucidation is needed to indicate that in such a combination of (primary) reaction spaces and auxiliary reactors the (primary) reaction spaces are operated batch-wise and the auxiliary reactors are operated continuously. In the system according to the invention the auxiliary reactor operates faster than in the usual application of an up-flow reactor because the effluent from the (primary) reaction space different from the known method for dispersions that have a low viscosity and a small percentage of solids, does not contain colloidal particles of slowly digestable materials.

From the time the methane generation in the primary reaction space occurs at a steady self-supporting rate, the operation in the auxiliary reaction is preferably discontinued.

The compost material obtained as a sludge from the reaction space possesses such a C:N ratio that it is substantially free of any smell and contains the food salts that were present in the solid organic starting material.

Example I

In a Fläkt device, big parts, such as wood, shoes, tires etc. were removed from a domestic waste.

Further a major part of the metallic waste, paper and plastics were removed from said domestic waste.

The remaining part was a wet crumbly mass that passed quantitatively a sieve having a mesh gauge of 10 mm. An analysis showed that it had the following composition:

| Water | 47,6% by weight |
|---|---|
| Ether extract | 1,6% by weight |
| Water extract | 7,4% by weight |
| Insoluble protein | 1,6% by weight |
| Pectin | 0,3% by weight |
| Hemi-cellulose | 1,9% by weight |
| Cellulose | 7,1% by weight |
| Lignin | 4,2% by weight |
| Ash | 28,1% by weight |
| | 99,8% by weight |

From said analysis it may be calculated that 1 kg of said domestic waste had a COD of 359 g/kg.

100 kg of said pretreated waste were blended with 5 kg anaerobic rotted down domestic waste having a moisture content of 48 weight %. Said blend was supplied to a primary reaction space, provided with a sieve bottom. Then water of 35°C was supplied to said reaction space till the water level in the reaction space was about 10 cm above the domestic waste level. The effluent was collected under the sieve bottom of the reaction space in a vessel having a capacity of 50 liters. Said effluent had the first day a COD value of 32 g/l, and in the subsequent days said COD values had a course as indicated in Fig. 3.

Said liquid was pumped from said 50 liter vessel into the lower side of an auxiliary reactor (methane reactor) at a rate of 71 l./day. Said methane reactor had a capacity of 200 liters. By

heating of the auxiliary reactor's sheath its contents was kept at a temperature of 35°C.

At the top said auxiliary reactor was provided with a separator for separating bacteria sludge, effluent (water) and biogas.

The separated bacteria-sludge was fed back in a usual way into the auxiliary reactor. The biogas was fed to a gasholder. Due to the fact that the auxiliary reactor (methane reactor) had been applied sufficiently high, the effluent could be fed under the influence of the gravitation to the primary reaction space (at a rate of 71 l./day).

Due to the feed of 71 liters of effluent from the primary reaction space to the auxiliary reactor, the first day an amount of biogas was produced that contained totally 825 liters of methane. The amount of biogas produced in a period of 10 days in the auxiliary reactor contained totally 6417 liters methane. Said methane production had a course as indicated in Fig. 4. After 10 days the supply of liquid to the auxiliary reactor was finished.

After 3 days a production of biogas in the primary reaction space started. The total amount of methane produced had a course as indicated in Fig. 4. The biogas produced within 30 days in the primary reaction space contained totally 2140 liters methane.

After 30 days the domestic waste had been rotted down to such a level that it could be used as a compost. 77,8 kg of compost were obtained. It had a percentage of moisture of 49,2 weight %, a percentage of ash of 39 weight %, and a percentage of organic material of 10,9 weight %.

Example II

The walls and the bottom of a reaction space having a surface of 10×5 m. and a height of 1 m. were coated internally with a plastic foil. On the bottom foil of said reaction space a system of drainage pipes was applied. Subsequently on said drainage system a sand layer having a thickness of 60 cm. was applied. On said sand layer 100 m$^3$ of worn-out tomato plants were dumped.

Said plant material had the following average composition:

| | |
|---|---|
| moisture | 84.8 % |
| ether extract | 0.43% |
| water extract | 2.17% |
| insoluble protein | 1.26% |
| pectin | 1.07% |
| hemicellulose | 0.70% |
| cellulose | 3.31% |
| lignin | 1.27% |
| ash | 4.54% |
| Totally | 99.55% |

From said composition may be calculated that 1 kg. of said plant material has a COD of 130 g./kg.

On the pile of tomato plants formed a system of sprinkle tubes was applied. Subsequently over said pile a plastic tilt was applied that formed a gas tight junction with the foil that coated the bottom and the wall of the reaction space. Effluent of a methane reactor having a temperature of 35°C was sprinkled through said sprinkle tubes over said pile at a capacity of 1500 l./h.

The permeate was drawn off from said reaction space through a drainage system. Said permeate had after the first day a COD-value of 3.7 g./l. Said liquid was fed to a vessel having a capacity of 10.000 l. and was heated therein up to a temperature of 35°C. Said heated liquid was fed at a rate of 1500 l./h. to the lower part of a methane reactor having a capacity of 10 m$^3$.

In said reactor, the organic materials were converted in the usual way into biogas by means of anaerobic bacteria at a temperature of 35°C.

Biogas and purified water were removed separately at the upper side of said reactor. The water was pumped to the sprinkle tubes in the reaction space. The above liquid stream from the reaction space to the methane reactor was maintained for 30 days. After said 30 days in the reaction space an amount of organic solids having a COD-value of 1240 kg. had been dissolved and removed with the permeate. Totally 750 m$^3$ biogas having a methane percentage of 60% were formed and removed from said amount of organic material. After said 30 days the tomato plants were sufficiently rotten down to be used as a compost. 13 m$^3$ compost having a solid percentage of 15 weight % were obtained.

**Claims**

1. A method for the anaerobic digestion of solid organic material originating from plants or animals or both, comprising the steps of:

a) simultaneously decomposing said solid organic waste under anaerobic conditions in a primary reaction space provided with a strainer while;

b) rinsing the solid organic waste from step (a) with water that may be polluted with soluble and/or insoluble inorganic and/or organic materials originating from plants and/or animals, to substantially dissolve and remove soluble organic and inorganic substances and the water soluble fatty acids formed by the decomposition;

c) feeding the liquid obtained in step (b) substantially free from undissolved solid organic waste from the primary reaction space into an auxiliary reactor;

d) leaving the remaining residu behind in the primary reaction space;

e) under anaerobic conditions in the auxiliary reactor, converting the dissolved organic material in the aqueous solution into a mixture of carbon dioxide and methane gas; and

f) removing the remaining residu from the primary reaction space.

2. The method according to claim 1 characterised in that the primary reaction space is fully sealed.

3. The method according to claim 1 or 2 characterised in that more than one primary reaction space is used.

4. The method according to claims 1—3 characterised in that the waste is delivered intermittently into the primary reaction space.

5. The method according to claims 1—4 characterised in that effluent from the auxiliary reactor is used in rinsing the decomposing solid organic waste in step (b).

6. The method according to claims 1—5 characterised in that step (b) is discontinued from the time the methane generation in the primary reaction space occurs at a steady self-supporting rate.

## Revendications

1. Procéde pour la digestion anaérobie d'une matière organique solide provenant de végétaux ou d'animaux ou des deux, comprenant les étapes qui consistent à:

a) décomposer simultanément les dits déchets organiques solides dans des conditions anaérobies dans un espace réactionnel primaire muni d'un filtre, tout en;

b) rinçant les déchets organiques solides de l'étape (a) avec de l'eau qui peut être poluée avec des matières minérales et/ou organiques solubles et/ou insolubles provenant de végétaux et/ou d'animaux, afin de dissoudre et d'éliminer sensiblement les substances organiques et inorganiques solubles et les acides gras hydrosolubles formés par la décomposition;

c) charger le liquide obtenu dans l'étape (b) sensiblement exempt de déchets organiques solides non dissous depuis l'espace de réaction primaire dans un réacteur auxiliaire;

d) abandonner le résidu restant dans l'espace réactionnel primaire;

e) dans des conditions anaérobies dans le réacteur auxiliaire, convertir la matière organique dissoute dans la solution aqueuse en un mélange de dioxyde de carbone et de gaz méthane; et

f) retirer le résidu restant de l'espace réactionnel primaire.

2. Procédé selon la revendication 1, caractérisé en ce que l'espace réactionnel primaire est entièrement hermétique.

3. Procédé selon la revendication 1 ou 2, caractérisé en ce que l'on utilise plus d'un espace réactionnel primaire.

4. Procédé selon les revendications 1—3, caractérisé en ce que les déchets sont introduits par intermittence dans l'espace réactionnel primaire.

5. Procédé selon les revendications 1—4, caractérisé en ce que l'on utilise l'effluent de réacteur auxiliaire pour rincer les déchets organiques solides en décomposition dans l'étape (b).

6. Procédé selon les revendications 1—5, caractérisé en ce que l'étape (b) est interrompue à partie du moment où le dégagement de méthane dans l'espace réactionnel primaire se produit à une vitesse régulière auto-entretenue.

## Patentansprüche

1. Verfahren zur anaeroben Digestion von festem organischen Material, das von Pflanzen oder Tieren oder beiden stammt, umfassend folgende Stufen:

a) gleichzeitige Zersetzung des festen organischen Abfalls unter anaeroben Bedingungen in einem primären Reaktionsraum, der mit einem Sieb ausgerüstet ist, währenddessen;

b) Spülen des festen organischen Abfalls aus der Stufe a) mit Wasser, das mit löslichen und/oder unlöslichen anorganischen und/oder organischen Materialien, die von Pflanzen und/oder Tieren stammen, verunreinigt sein kann, um lösliche organische und anorganische Substanzen und die wasserlöslichen Fettsäuren, die durch die Zersetzung gebildet werden, im wesentlichen aufzulösen und zu entfernen;

c) Beschicken der in der Stude b) erhaltenen Flüssigkeit, die im wesentlichen frei von nichtgelöstem festen organischen Abfall ist, aus dem primären Reaktionsraum in einen Hilfsreaktor;

d) Zurücklassen des verbleibenden Rückstands in dem primären Reaktionsraum;

e) Umwandeln des gelösten organischen Materials in der wässerigen Lösung, unter anaeroben Bedingungen in dem Hilfsreaktor, in ein Gemisch aus Kohlendioxid und Methangas; und

f) Entfernen des verbleibenden Rückstands aus dem primären Reaktionsraum.

2. Verfahren nach Anspruch 1, dadurch gekennzeichnet, dass der primäre Reaktionsraum völlig verschlossen ist.

3. Verfahren nach Anspruch 1 oder 2, dadurch gekennzeichnet, dass mehr als ein primärer Reaktionsraum verwendet wird.

4. Verfahren nach den Ansprüchen 1—3, dadurch gekennzeichnet, dass der Abfall intermittierend in den primären Reaktionsraum eingeführt wird.

5. Verfahren nach den Ansprüchen 1—4, dadurch gekennzeichnet, dass der Abfluss aus dem Hilfsreaktor verwendet wird um den sich in der Stufe b) zersetzenden festen organischen Abfall zu spülen.

6. Verfahren nach den Ansprüchen 1—5, dadurch gekennzeichnet, dass die Stufe b) von dem Zeitpunkt an, zu dem die Methanerzeugung in dem primären Reaktionsraum in einem beständigen, selbsterhaltenden Ausmass erfolgt, nicht mehr weitergeführt wird.

## Fig-1

## Fig-2

## Fig-3

Fig-4